# EUROPÄISCHE PATENTSCHRIFT

(11) **EP 0 863 727 B1**
(45) Veröffentlichungstag und Bekanntmachung des Hinweises auf die Patenterteilung: **17.01.2001**
(21) Anmeldenummer: 95936924.0
(22) Anmeldetag: 30.11.1995
(51) Int. Cl.: A61B 17/70

(54) **VORRICHTUNG ZUR KNOCHENFIXATION**
BONE-FIXING DEVICE
DISPOSITIF D'OSTEOSYNTHESE

(43) Veröffentlichungstag der Anmeldung: 16.09.1998
(73) Patentinhaber: SYNTHES AG Chur, 7002 Chur (CH)
(72) Erfinder: SCHLAEPFER, Fridolin, CH-8750 Glarus (CH); HATEBUR, Alexander, CH-4056 Basel (CH)
(74) Vertreter: Lusuardi, Werther Giovanni, Dr.
(86) Internationale Anmeldenummer: CH9500281
(87) Internationale Veröffentlichungsnummer: WO9719646

(56) Entgegenhaltungen:
- US-A- 4 289 123

## Beschreibung

Die Erfindung betrifft eine Vorrichtung zur Knochenfixation gemäss dem Oberbegriff des Patentanspruchs 1.

Für die anteriore Fusion von Wirbelsegmenten (Frakturen, Tumore, Infektionen, kongenitale und posttraumatische Kyphosen, Deformitäten) stehen eine grosse Anzahl von Platten- wie auch Stab-Systemen zur Verfügung.
Grosse Vorteile der Plattensysteme, z.B. gemäss der PCT/US93/08433 (vgl. WO-A-9406360), sind die relativ kleinen Dimensionen und die im allgemeinen einfache Handhabung. Gewichtige Nachteile diese bekannten Plattensysteme bestehen darin, dass der Fusionsbereich nicht aktiv unter Kompression gesetzt werden kann, das grosse Sortiment an teuren Plattenimplantaten, und im Fall von Deformitäten, keine Korrekturmöglichkeiten.
Zwar haben die bekannten Stab-Systeme, z.B. gemäss PCT/US93/10908 (vgl. WO-A-9410927) oder PCT/EP94/01606 (vgl. WO-A-9426194), die oben aufgeführten Nachteile der Plattensysteme nicht mehr, dafür sind sie aber von Natur aus in den Dimensionen höher und schwieriger zum Handhaben.
Aus der US 4,289,123 ist bereits eine Knochenfixationsvorrichtung gemäss dem Oberbegriff des Patentanspruchs 1 bekannt, welche allerdings durch die gewählte Anordnung ihrer Elemente ebenfalls überdimensioniert und sperrig ist.

Hier will die Erfindung Abhilfe schaffen. Der Erfindung liegt die Aufgabe zugrunde, eine Vorrichtung zur Knochenfixation zu schaffen, welche die Vorteile der bekannten Platten- und Stab-Systeme vereinigt, d.h. eine einfache Handhabung, plattenähnliche Dimensionen, ein kostengünstiges Sortiment, sowie Komprimier- und Korrekturmöglichkeiten bietet.

Zur Lösung dieser Aufgabe ist die eingangs genannte Anordnung durch die Merkmale des kennzeichnenden Teils des unabhängigen Anspruchs 1 weitergebildet.

Für die Anwendung am Patienten werden zwei erfindungsgemässe komplementäre Vorrichtungen an zwei parallelen Längsträger befestigt, vgl. Anspruch 16. Jede Vorrichtung ist mit zwei Knochenschrauben mit dem entsprechenden Wirbelkörper verbunden. Die Knochenschrauben weisen zweckmässigerweise ein Knochen- und ein Maschinengewinde auf. Die beiden Gewinde sind so konzipiert, dass sie die gleiche Steigung aufweisen und nahtlos von einem zum anderen übergehen. Die Plattenlöcher in den beiden Vorrichtungen weisen das gleiche Maschinengewinde wie die Knochenschrauben auf. Die Geometrie des Gewindes im Knochenplattenteil der Vorrichtung ist dabei derart gestaltet, dass nach dem Anziehen der Schrauben die Vorrichtung und die Schraube winkelstabil und vibrationssicher miteinander verbunden miteinander sind.
Die Längsträger sind direkt mit den beiden erfindungsgemässen Vorrichtungen und nicht mit den Knochenschrauben verbunden. Dazu weisen die Backenteile der erfindungsgemässen Vorrichtungen zweckmässigerweise je ein Sackloch und eine Durchgangsloch auf, in welche die beiden Längsträger eingeführt und mittels Stellschrauben fixiert werden können. Die Löcher für die Längsträger sind derart angeordnet, dass das Sackloch auf dem. Backenteil der einen Vorrichtung mit dem Durchgangsloch auf dem Backenteil der anderen Vorrichtung fluchtet und die Knochenschrauben auch nach der Montage der Längsträger noch eingebracht werden können. Diese spezielle Anordnung und Ausführung der Löcher für die Längsträger führen zu all den oben erwähnten Vorteilen der erfindungsgemässen Kombination, wie einfache Handhabung, kleine Dimensionen und unbegrenztes in situ Distrahieren/Komprimieren.

Die Erfindung und Weiterbildungen der Erfindung werden im folgenden anhand der teilweise schematischen Darstellungen mehrerer Ausführungsbeispiele noch näher erläutert.
Es zeigen:
Fig. 1 eine perspektivische Darstellung einer ersten erfindungsgemässen Vorrichtung;
Fig. 2 eine perspektivische Darstellung einer zweiten zur ersten komplementären erfindungsgemässen Vorrichtung;
Fig. 3 eine perspektivische Darstellung der beiden erfindungsgemässen Vorrichtungen nach Fig. 1/2, welche auf zwei Längsträgern montiert sind;
Fig. 4 eine perspektivische Darstellung einer ersten Modifikation einer erfindungsgemässen Vorrichtung mit einem noch nicht eingeführten Längsträger;
Fig. 5 eine perspektivische Darstellung einer zweiten Modifikation einer erfindungsgemässen Vorrichtung mit einem noch nicht eingeführten Längsträger;
Fig. 6 einen Längsschnitt durch eine Plattenloch mit Gewinde;
Fig. 7 einen Längsschnitt durch ein kalottenförmiges Plattenloch.

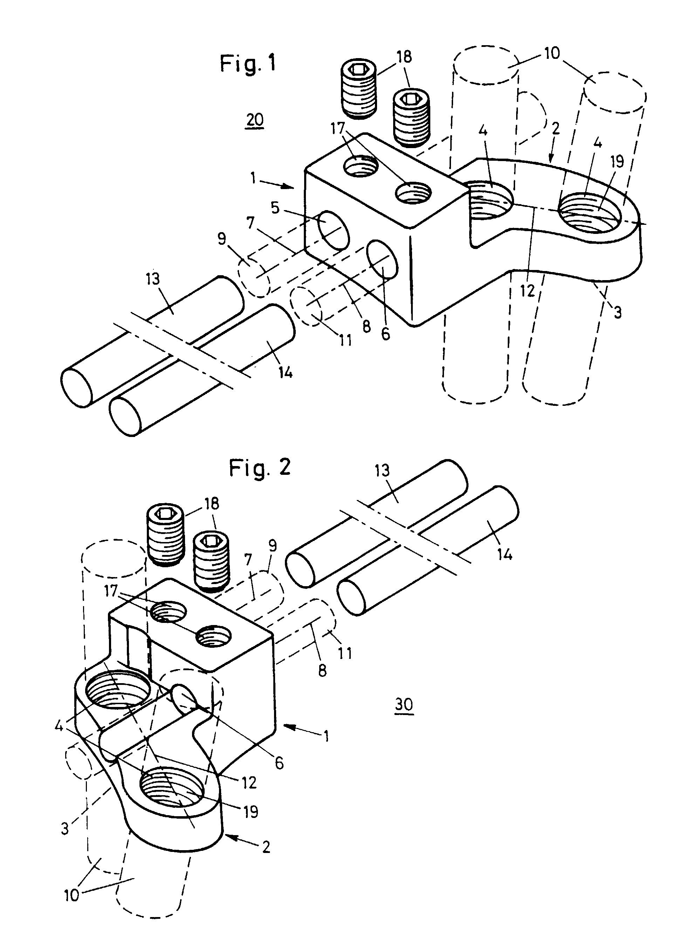

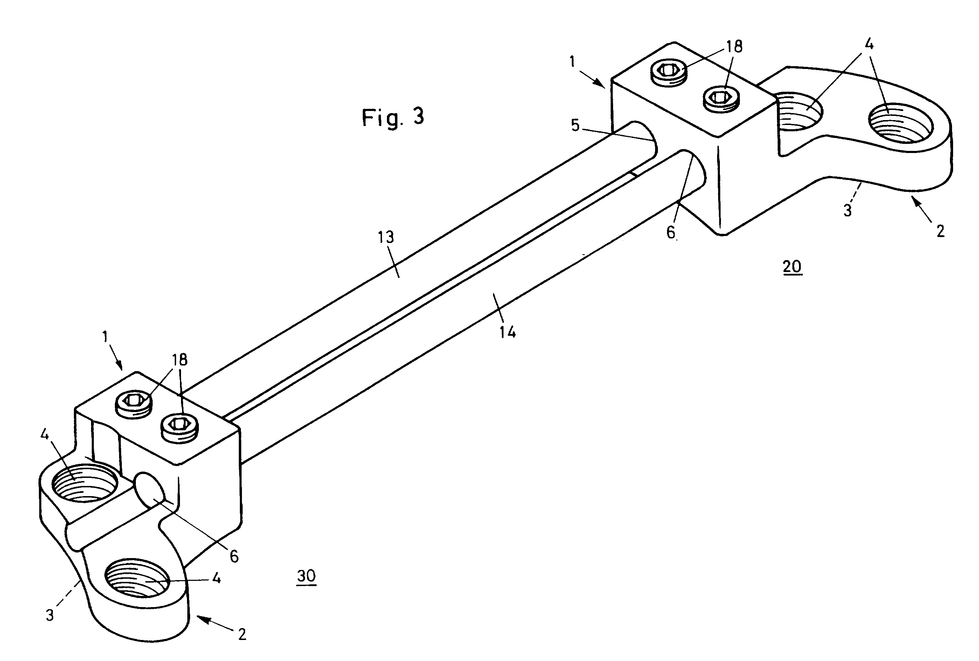

Die in Fig. 1 dargestellte erfindungsgemässe Vorrichtung 20 zur Knochenfixation besteht im wesentlichen aus einer Backe 1 und einer daran anschliessenden Knochenplatte 2 mit einer Knochenkontaktfläche 3 und zwei die Knochenplatte 2 durchquerende Plattenlöcher 4, deren Zentren auf einer gemeinsamen Verbindungslinie 12 liegen, zur Aufnahme eines Knochenfixationsmittels, beispielsweise einer Knochenschraube. Die Backe 1 besitzt einen ersten Führungskanal 5 zur Aufnahme eines ersten Längsträgers 13 und einen zweiten Führungskanal 6 zur Aufnahme eines zweiten Längsträgers 14. Die beiden Führungskanäle 5,6 verlaufen parallel zueinander, wobei die Achsen 7,8 der beiden Führungskanäle 5,6 in einer zur Knochenkontaktfläche 3 parallel verlaufenden Ebene liegen. Der vom ersten Führungskanal 5 definierte Kreiszylinder 9 schneidet die von den Plattenlöchern 4 je definierten Kreiszylinder 10 nicht, währenddem der vom zweiten Führungskanal 6 definierte Kreiszylinder 11 einen von den Plattenlöchern 4 je definierten Kreiszylinder 10 schneidet (Fig. 1 und 3).

Der erste Führungskanal 5 durchdringt die Backe 1 völlig, so dass ein darin eingeführter Längsträger 13 in jede beliebige axiale Position geschoben werden kann. Hingegen ist der zweite Führungskanal 6 als Sackloch ausgebildet, welches sich auf der, der Knochenplatte 2 abgewendeten Seite öffnet. Ein Längsträger 14 kann somit nur bis in die maximale Tiefe des Sacklochs geschoben werden und nicht in den Bereich der Kreiszylinder 10 der Plattenlöcher 4 vordringen. Die beiden Führungskanäle 5,6 verfügen über senkrecht zu Ihren Achsen 7,8 verlaufende Bohrungen 17 zur Aufnahme von Fixationsmitteln 18 (in Form von Stellschrauben) für die Längsträger 13,14. Die Verbindungslinie 12, welche die beiden Plattenlöcher 4 verbindet, schliesst einen positiven Winkel von +30° mit den Achsen 8,9 der beiden Führungskanäle 5,6 ein. Dieser Winkel kann im Bereich von +10° bis +50°, vorzugsweise von +20° bis +40°, variieren.
Die Knochenkontaktfläche 3 bildet ein Zylindersegment mit einem Zylinderradius von 30 mm, wobei die Achse des Zylindersegmentes parallel zu den Längsachsen 9,11 der Führungskanäle 5,6 verläuft. Der Zylinderradius kann innerhalb eines Bereichs von 25 und 35 mm, vorzugsweise von 29 - 31 mm variieren. Die Längsachsen der Plattenlöcher 4 liegen in Parallelebenen, die senkrecht zur Längsachse der zylinderabschnittförmigen Knochenkontaktfläche 3 stehen.

In Fig. 2 ist eine zur Vorrichtung 20 komplementäre Vorrichtung 30 dargestellt, welche sich insbesondere dadurch unterscheidet, dass bei ihr die Verbindungslinie 12 einen negativen Winkel von - 30° mit den Achsen 8,9 der beiden Führungskanäle 5,6 einschliesst. Dieser Winkel kann im Bereich von -10° bis - 50°,vorzugsweise von -20° bis -40°, variieren.

Wie in Fig. 3 dargestellt können die beiden Vorrichtungen 20 und 30 mit zwei Längsträgern 13,14 zu einer Kombinationsvorrichtung ergänzt werden, wobei ein erster Längsträger 13 im ersten Führungskanal 5 der einen Vorrichtung 20 und im zweiten Führungskanal 6 der anderen Vorrichtung 30 eingeführt wird und ein zweiter Längsträger 14 im zweiten Führungskanal 6 der einen Vorrichtung 20 und im ersten Führungskanal 5 der anderen Vorrichtung 30 eingeführt wird.
Der Abstand der beiden Vorrichtungen 20,30 zueinander ist durch Verschiebung auf den beiden Längsträgern 13,14 einstellbar und kann mittels der Fixationsmittel 18 in Form von Stellschrauben in jeder beliebigen Lage fixiert werden.

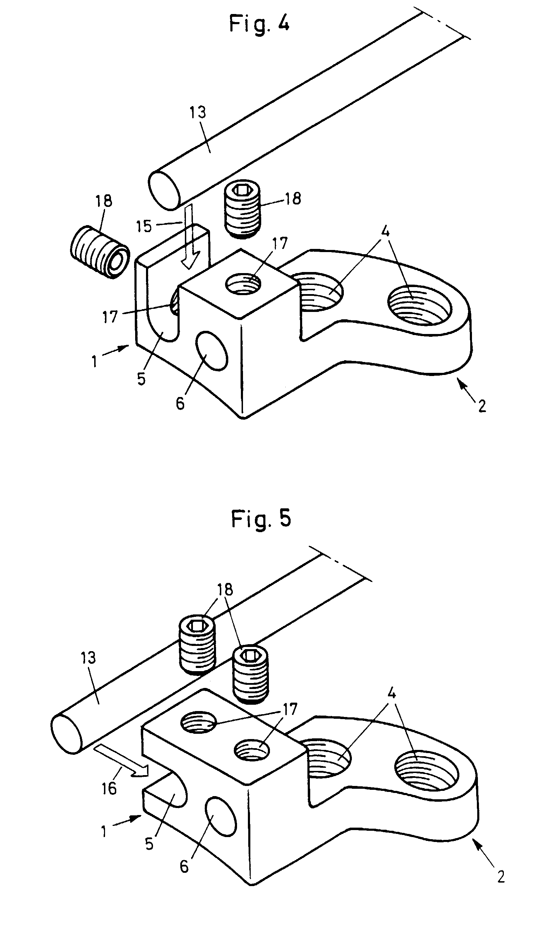

Wie in Fig. 4 dargestellt, kann die Vorrichtung 20 derart modifiziert werden, dass der erste Führungskanal 5 auf der, der Knochenkontaktfläche 3 abgewandten Seite offen ausgebildet ist, so dass ein Längsträger 13 von oben - in Richtung des Pfeiles 15 - darin einlegbar ist.

Eine weitere Ausführungsform der Vorrichtung 20 ist in Fig. 5 dargestellt und besteht darin, dass der erste Führungskanal 5 in Richtung seiner benachbarten Seitenfläche 24 der Backe 1 offen ausgebildet ist, so dass ein Längsträger 13 von der Seite - in Richtung des Pfeiles 16 - darin einlegbar ist.

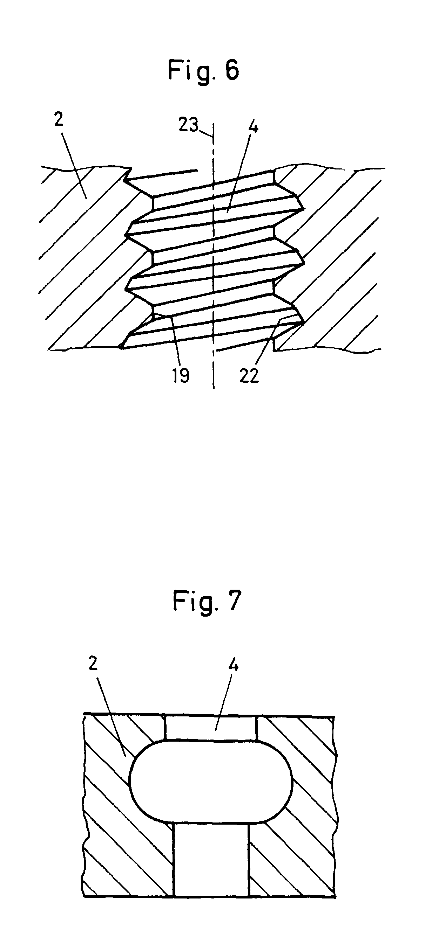

Wie in Fig. 6 dargestellt können die Plattenlöcher 4 auch ein mehrgängiges Gewinde 19 aufweisen, welches eine entsprechend ausgebildete - im Kopfbereich ein Aussengewinde aufweisende - Knochenschraube aufnehmen kann, so dass eine rigide und axial ausgerichtete Verbindung zwischen Knochenschraube und Knochenplatte 2 resultiert. Das Gewinde 19 weist einen flächigen Gewindegrund 22 auf, welcher zusammen mit der Gewindelängsachse 23 einen Winkel von 30° bildet. Der Winkel kann in einem Bereich zwischen 20° und 40°, vorzugsweise von 29° - 31 ° variieren.

Die Plattenlöcher 4 können auch wie in Fig. 7 dargestellt kalottenförmig ausgebildet sein, um eine geschlitzte Kugel mit einer konischen Bohrung für die Knochenschraube aufnehmen zu können, so dass die Knochenschraube in einem bestimmten Winkel zur Knochenplatte 2 eingestellt und winkelstabil fixiert werden kann.

Im folgenden wird der Ablauf der operativen Applikation der erfindungsgemässen Vorrichtung im Detail beschrieben.
Für oder nach der Behandlung der anterioren Läsion werden die betroffenen Wirbelkörper mit einem Wirbelspreizer gespreizt und Knochenspäne, bzw. Wirbelersatzkörper in den entstandenen Defekt eingeführt. Danach wird die Länge der zu benutzenden Längsträger 13,14 bestimmt. In jedem der zweiten Führungskanäle 6, welche als Sackloch ausgebildet sind, je einer erfindungsgemässen Vorrichtung 20,30 wird ein Längsträger 13;14 eingeführt und mittels einer Stellschraube 17 fixiert.
Die mit je einem Längsträger 13, bzw. 14 instrumentierte erfindungsgemässen Vorrichtungen 20 und 30 werden nun wie in Fig. 3 dargestellt zu einem Kombinationsvorrichtung zusammengesetzt. Dies geschieht dadurch, dass die freien Enden der Längsträger 13,14 in den ersten Führungskanal 5, der jeweils anderen erfindungsgemässen Vorrichtung 20, bzw. 30 eingeschoben wird und mit einer Stellschraube 18 leicht fixiert wird.
Jede der beiden - nun miteinander verbundenen - erfindungsgemässen Vorrichtungen 20,30 wird nun mit einem (zeichnerisch nicht dargestellten) Implantathalter, z.B. einer Bohrhülse mit integrierter Ahle, in den Plattenlöchern 4 instrumentiert. Die Kombinationsvorrichtung wird nun mit den Implantathaltern seitlich auf die Wirbelsäule gepresst, die erste Bohrahle wird entsichert, ein Loch gebohrt, die Bohrhülse mit der integrierten Bohrahle entfernt und eine Knochenschraube durch das Plattenloch 4 in das im Knochen gebohrte Loch eingedreht. Danach wird die zweite Bohrahle entsichert, ein Loch gebohrt, die Bohrhülse mit der integrierten Bohrahle entfernt und eine Knochenschraube in das gebohrte Loch eingedreht.
Solange nur je eine Knochenschraube in die einzelnen Wirbelkörper eingedreht ist, kann immer noch kyphosiert oder lordosiert werden.
Nun werden die Bohrhülsen mit integrierter Ahle in die verbliebenen Plattenlöcher 4 eingedreht und wie oben beschrieben verfahren.
Die mittels der Kombinationsvorrichtung fusionierte Zone lässt sich nun dadurch komprimieren, dass die Stellschrauben 18, welche die beiden Längsträger 13,14 in den ersten Führungskanälen 5 fixieren, gelöst werden und die Backen 1 der beiden erfindungsgemässen Vorrichtungen 20,30 mit einer Kompressionszange zusammengeschoben werden.

## Patentansprüche

1. Vorrichtung zur Knochenfixation (20;30) bestehend aus einer Backe (1) und einer daran anschliessenden Knochenplatte (2) mit einer Knochenkontaktfläche (3) und mindestens einem die Knochenplatte (2) durchquerenden Plattenloch (4) zur Aufnahme eines Knochenfixationsmittels, wobei
A) die Backe (1) einen ersten Führungskanal (5) zur Aufnahme eines Längsträger besitzt;
B) die Backe (1) einen zweiten Führungskanal (6) zur Aufnahme eines Längsträger besitzt;
C) die beiden Führungskanäle (5,6) parallel zueinander verlaufen;
D) die Achsen (7,8) der beiden Führungskanäle (5,6) in einer zur Knochenkontaktfläche (3) parallel verlaufenden Ebene liegen; und
E) der vom ersten Führungskanal (5) definierte Kreiszylinder (9) den vom Plattenloch (4), bzw. die von den Plattenlöchern (4) je definierten Kreiszylinder (10) nicht schneidet;
**dadurch gekennzeichnet, dass**
F) der vom zweiten Führungskanal (6) definierte Kreiszylinder (11) den vom Plattenloch (4), bzw. einen von den Plattenlöchern (4) je definierten Kreiszylinder (10) schneidet.

2. Vorrichtung nach Anspruch 1, dadurch gekennzeichnet, dass der erste Führungskanal (5) die Backe (1) völlig durchdringt.

3. Vorrichtung nach Anspruch 1 oder 2, dadurch gekennzeichnet, dass der zweite Führungskanal (6) als Sackloch ausgebildet ist, welches sich auf der, der Knochenplatte (2) abgewendeten Seite öffnet.

4. Vorrichtung nach einem der Ansprüche 1 bis 3, dadurch gekennzeichnet, dass die Knochenplatte (2) mindestens zwei Plattenlöcher (4) besitzt, deren Zentren auf einer gemeinsamen Verbindungslinie (12) liegen.

5. Vorrichtung nach einem der Ansprüche 1 bis 4, dadurch gekennzeichnet, dass mindestens einer der Führungskanäle (5,6) auf der, der Knochenkontaktfläche (3) abgewandten Seite offen ausgebildet ist, so dass ein Längsträger (13) von oben (15) darin einlegbar ist.

6. Vorrichtung nach einem der Ansprüche 1 bis 4, dadurch gekennzeichnet, dass mindestens einer der Führungskanäle (5,6) in Richtung seiner benachbarten Seitenfläche (24) der Backe (1) offen ausgebildet ist, so dass ein Längsträger (13) von der Seite (16) darin einlegbar ist.

7. Vorrichtung nach einem der Ansprüche 1 bis 6, dadurch gekennzeichnet, dass die beiden Führungskanäle (5,6) über senkrecht zu Ihren Achsen (7,8) verlaufenden Bohrungen (17) verfügen zur Aufnahme von Fixationsmitteln (18) für die Längsträger (13,14).

8. Vorrichtung nach einem der Ansprüche 4 bis 7, dadurch gekennzeichnet, dass die Verbindungslinie (12) einen Winkel im Bereich von +10° bis +50°, vorzugsweise von +20° bis +40° mit den Achsen (8,9) der beiden Führungskanäle (5,6) einschliesst.

9. Vorrichtung nach einem der Ansprüche 4 bis 7, dadurch gekennzeichnet, dass die Verbindungslinie (12) einen Winkel im Bereich von -10° bis -50°, vorzugsweise von -20° bis -40° mit den Achsen (8,9) der beiden Führungskanäle (5,6) einschliesst.

10. Vorrichtung nach einem der Ansprüche 1 bis 9, dadurch gekennzeichnet, dass die Knochenkontaktfläche (3) ein Zylindersegment bildet mit einem Zylinderradius zwischen 25 und 35 mm, vorzugswiese 29 - 31 mm, wobei die Achse des Zylindersegmentes parallel zu den Längsachsen (9,11) der Führungskanäle (5,6) verläuft.

11. Vorrichtung nach einem der Ansprüche 1 bis 10, dadurch gekennzeichnet, dass die Längsachsen der Plattenlöcher (4) in Parallelebenen liegen, die senkrecht zur Längsachse der zylinderabschnittförmigen Knochenkontaktfläche (3) stehen.

12. Vorrichtung nach einem der Ansprüche 1 bis 11, dadurch gekennzeichnet, dass die Plattenlöcher (4) ein, vorzugsweise mehrgängiges Gewinde (19) aufweisen.

13. Vorrichtung nach Anspruch 12, dadurch gekennzeichnet, dass Gewinde (19) einen flächigen Gewindegrund (22) aufweist.

14. Vorrichtung nach Anspruch 13, dadurch gekennzeichnet, dass der flächige Gewindegrund (22) des Gewindes (19) zusammen mit der Gewindelängsachse (23) einen Winkel zwischen 20° und 40°, vorzugsweise von 29° - 31° bildet.

15. Vorrichtung nach einem der Ansprüche 1 bis 14, dadurch gekennzeichnet, dass die Plattenlöcher (4) kalottenförmig ausgebildet sind.

16. Kombination von zwei zueinander komplementären Vorrichtungen (20,30) nach einem der Ansprüche 1 - 15, dadurch gekennzeichnet, dass
A) ein erster Längsträger (13) im ersten Führungskanal (5) der einen Vorrichtung (20) und im zweiten Führungskanal (6) der anderen Vorrichtung (30) einführbar ist;
B) ein zweiter Längsträger (14) im zweiten Führungskanal (6) der einen Vorrichtung (20) und im ersten Führungskanal (5) der anderen Vorrichtung (30) einführbar ist;
C) der Abstand der beiden Vorrichtungen (20,30) zueinander durch Verschiebung auf den beiden Längsträgern (13,14) einstellbar ist;
D) die Kreiszylinder (9,11;10) der zu den zwei Längsträgern (13,14) korrespondierenden Führungskanäle (5,6) und Plattenlöcher (4) der beiden zueinander komplementären Vorrichtungen (20,30) so zusammenwirken, dass die beiden Längsträger (13,14) in der jeweils gegenseitigen Vorrichtung gleiten können; wobei
E) auch nach Montage der Kombination, Knochenschrauben (18) in die Plattenlöcher (4) einführbar sind.

## Claims

1. Device (20;30) for the fixation of bones consisting of a block (1) and a bone plate (2) connected thereto with a bone-contact surface (3) and at least one plate hole (4) passing through the bone plate (2) in order to accommodate a bone-fixing means, whereby
A) the block (1) comprises a first guide channel (5) in order to accommodate a longitudinal carrier;
B) the block (1) comprises a second guide channel (6) in order to accommodate a longitudinal carrier;
C) the two guide channels (5;6) are running parallel to each other;
D) the axes (7;8) of the two guide channels (5;6) are lying in a plane which is parallel to the bone contact surface (3); and
E) the circular cylinder (9) defined by the first guide channel (5) does not intersect the circular cylinders (10) each defined by the plate hole (4) or the plate holes (4) respectively,
characterized in that
F) the circular cylinder (11) defined by the second guide channel (6) intersects the circular cylinders (10) each defined by the plate hole (4) or the plate holes (4) respectively.

2. Device according to claim 1, characterized in that the first guide channel (5) completely penetrates the block (1).

3. Device according to claim 1 or 2, characterized in that the second guide channel (6) is shaped as a pocket hole open on the side opposite to the bone plate (2).

4. Device according to one of the claims 1 to 3, characterized in that the bone plate (2) comprises at least two plate holes (4) whose centres lie on a common connecting line (12).

5. Device according to one of the claims 1 to 4, characterized in that at least one of the guide channels (5;6) is shaped open at the side opposite to the bone-contact surface (3) so that a longitudinal carrier (13) is insertable therein from above (15).

6. Device according to one of the claims 1 to 4, characterized in that at least one of the guide channels (5;6) is shaped open in the direction of its adjacent side surface (24) of the block (1) so that a longitudinal carrier (13) is insertable therein from the side (16).

7. Device according to one of the claims 1 to 6, characterized in that the two guide channels (5;6) provide bore holes (17) running orthogonal to their axes (7;8) in order ot accommodate fixation means (18) for the longitudinal carriers (13;14).

8. Device according to one of the claims 4 to 7, characterized in that the connecting line (12) encloses an angle in the range of +10° to +50° preferably of +20° to +40° with the axes (7;8) of the two guide channels (5;6).

9. Device according to one of the claims 4 to 7, characterized in that the connection line (12) encloses an angle in the range of -10° to -50° preferably of -20° to -40° with the axes (7;8) of the two guide channels (5;6).

10. Device according to one of the claims 1 to 9, characterized in that the bone-contact surface (3) forms a cylinder segment with a cylinder radius between 25 and 35 mm preferably between 29 - 31 mm whereby the axis of the cylinder segment runs parallel to the longitudinal axes (7;8) of the guide channels (5;6).

11. Device according to one of the claims 1 to 10, characterized in that the longitudinal axes of the plate holes (4) lie within parallel planes which are perpendicular to the longitudinal axis of the bone-contact surface (3) shaped as a cylinder segment.

12. Device according to one of the claims 1 to 11, characterized in that the plate holes (4) provide a preferably multiple-threaded thread (19).

13. Device according to claim 12, characterized in that the thread (19) provides a planar thread bottom (22).

14. Device according to claim 13, characterized in that the planar thread bottom (22) of the thread (19) forms an angle between 20° and 40°, preferably 29° - 31° together with the longitudinal axis (23) of the thread.

15. Device according to one of the claims 1 to 14, characterized in that the plate holes (4) are formed spherical.

16. Combination of two devices according to one of the claims 1 to 15, characterized in that
A) a first longitudinal carrier (13) is insertable into the first guide channel (5) of the one device (20) and into the second guide channel (6) of the other device (30);
B) a second longitudinal carrier (14) is insertable into the second guide channel (6) of the one device (20) and into the first guide channel (5) of the other device (30), whereby
C) the distance between the two devices (20;30) to each other is adjustable through displacement on the two longitudinal carriers (13;14).

## Revendications

1. Dispositif (20 ; 30) pour la fixation d'os, constitué d'une joue (1) et d'une plaque pour os (2) qui s'y raccorde et qui est dotée d'une surface de contact (3) avec l'os et d'au moins un trou de plaque (4) qui traverse la plaque pour os (2), pour la réception d'un moyen de fixation de l'os, dans lequel :
A) la joue (1) possède un premier canal de guidage (5) pour la réception d'un support longitudinal ;
B) la joue (1) possède un deuxième canal de guidage (6) pour la réception d'un support longitudinal ;
C) les deux canaux de guidage (5, 6) s'étendent parallèlement l'un à l'autre ;
D) les axes (7, 8) des deux canaux de guidage (5, 6) sont situés dans un plan qui s'étend parallèlement à la surface de contact (3) avec l'os ; et
E) le cylindre circulaire (9) défini par le premier canal de guidage (5) ne coupe pas le cylindre circulaire (10) défini par le trou de plaque (4) ou les cylindres circulaires (10) définis chacun par les trous de plaque (4) ;
caractérisé en ce que
F) le cylindre circulaire (11) défini par le deuxième canal de guidage (6) coupe le cylindre circulaire (10) défini par le trou de plaque (4) ou l'un des cylindres circulaires (10) définis chacun par les trous de plaque (4).

2. Dispositif selon la revendication 1, caractérisé en ce que le premier canal de guidage (5) traverse complètement la joue (1).

3. Dispositif selon la revendication 1 ou 2, caractérisé en ce que le deuxième canal de guidage (6) est configuré comme trou aveugle qui s'ouvre sur le côté non tourné vers la plaque pour os (2).

4. Dispositif selon l'une des revendications 1 à 3, caractérisé en ce que la plaque pour os (2) possède au moins deux plaques (4) dont les centres sont situés sur une ligne de liaison (12) commune.

5. Dispositif selon l'une des revendications 1 à 4, caractérisé en ce qu'au moins l'un des canaux de guidage (5, 6) est configuré ouvert sur le côté non tourné vers la surface de contact (3) avec l'os, de sorte qu'un support longitudinal (13) puisse y être inséré par le haut (15).

6. Dispositif selon l'une des revendications 1 à 4, caractérisé en ce qu'au moins l'un des canaux de guidage (5, 6) est configuré ouvert en direction de sa surface latérale (24) voisine de la joue (1), de sorte qu'un support longitudinal (13) puisse y être inséré par le côté (16).

7. Dispositif selon l'une des revendications 1 à 6, caractérisé en ce que les deux canaux de guidage (5, 6) disposent d'alésages (17) qui s'étendent perpendiculairement par rapport à leurs axes (7, 8) pour la réception de moyens de fixation (18) des supports longitudinaux (13, 14).

8. Dispositif selon l'une des revendications 4 à 7, caractérisé en ce que la ligne de liaison (12) forme avec les axes (8, 9) des deux canaux de guidage (5, 6) un angle compris dans la plage + 10° à + 50°, de préférence de + 20° à + 40°.

9. Dispositif selon l'une des revendications 4 à 7, caractérisé en ce que la ligne de liaison (12) forme avec les axes (8, 9) des deux canaux de guidage (5, 6) un angle compris dans la plage - 10° à - 50°, de préférence de - 20° à - 40°.

10. Dispositif selon l'une des revendications 1 à 9, caractérisé en ce que la surface de contact (3) avec l'os forme un segment de cylindre avec un rayon de cylindre compris entre 25 et 35 mm, de préférence entre 29 et 31 mm, et l'axe du segment de cylindre s'étend parallèlement aux axes longitudinaux (9, 11) des canaux de guidage (5, 6).

11. Dispositif selon l'une des revendications 1 à 10, caractérisé en ce que les axes longitudinaux des trous de plaque (4) sont situés dans des plans parallèles qui sont perpendiculaires à l'axe longitudinal de la surface de contact (3) avec l'os en forme de tronçon de cylindre.

12. Dispositif selon l'une des revendications 1 à 11, caractérisé en ce que les trous de plaque (4) présentent un filet (19), de préférence à plusieurs pas.

13. Dispositif selon la revendication 12, caractérisé en ce que le filet (19) présente une base de filet (22) plane.

14. Dispositif selon la revendication 13, caractérisé en ce que la base plane de filet (22) du filet (19) forme avec l'axe longitudinal (23) du filet un angle compris entre 20° et 40°, de préférence entre 29° et 31°.

15. Dispositif selon l'une des revendications 1 à 14, caractérisé en ce que les trous de plaque (4) sont configurés en forme de calotte sphérique.

16. Combinaison de deux dispositifs (20, 30) complémentaires l'un de l'autre, selon l'une des revendications 1 à 15, caractérisée en ce que :
A) un premier support longitudinal (13) peut être inséré dans un premier canal de guidage (5) du premier dispositif (20) et dans le deuxième canal de guidage (6) de l'autre dispositif (30) ;
B) un deuxième support longitudinal (14) peut être inséré dans le deuxième canal de guidage (6) du premier dispositif (20) et dans le premier canal de guidage (5) de l'autre dispositif (30) ;
C) la distance mutuelle entre les deux dispositifs (20, 30) peut être ajustée par coulissement sur les deux supports longitudinaux (13, 14) ;
D) les cylindres circulaires (9, 11 ; 10) des canaux de guidage (5, 6) qui correspondent aux deux supports longitudinaux (13, 14) et des trous de plaque (4) des deux dispositifs (20, 30) complémentaires l'un de l'autre coopèrent de telle sorte que les deux supports longitudinaux (13, 14) peuvent glisser dans chaque dispositif opposé ; et
E) même après le montage de la combinaison, des vis pour os (18) peuvent être insérés dans les trous de plaque (4).
